# EUROPEAN PATENT SPECIFICATION

(11) **EP 0 644 177 B1**
(45) Date of publication and mention of the grant of the patent: **09.07.1997**
(21) Application number: 94202692.3
(22) Date of filing: 19.09.1994
(51) Int. Cl.: C07C 209/26

(54) **Process for the preparation of dibenzylamine**
Verfahren zur Herstellung von Dibenzylaminen
Procédé pour la préparation de dibenzylamine

(30) Priority: 20.09.1993 BE 9300975
(43) Date of publication of application: 22.03.1995
(73) Proprietor: DSM N.V., 6411 TE Heerlen (NL)
(72) Inventor: Castelijns, Anna Maria Cornelia Francisca, NL-67176 JB Beek (L.) (NL); Maas, Peter Johannes Dominicus, NL-6155 NA Schinnen (NL)

(56) References cited:
- WO-A-93/13047
- Z. PHYSIOL. CHEM., vol.107, 1927 pages 186 - 211 F. KNOOP ET AL. 'Beiträge zu Synthese und Abbau von Aminosäuren'

## Description

The invention relates to a process for preparation of dibenzylamine through the hydrogenation of benzaldehyde in the presence of ammonia and a catalyst containing palladium.

Such a process is known from WO-A-9313047.

In the known process the reaction is carried out in a homogenous solution of benzaldehyde in methanol. In order to safeguard a homogeneous solution relatively high amounts of solvents (850 wt% methanol calculated with respect to benzaldehyde) are used, which leads to a commercially less attractive process in view of e.g. low produciton capacities and high destillation costs. Moreover a relatively large amount of catalyst is used (2 wt.% calculated with respect to benzaldehyde).

The invention provides a process which does not have the above-mentioned disadvantages and with which dibenzylamine can be obtained with a high selectivity (>90%) with complete conversion of the benzaldehyde in a particularly attractive commercial process.

According to the invention this is accomplished in that the reaction is performed in the presence of a solvent or dispersion medium in an amount of 3-60 wt.% calculated with respect to benzaldehyde.

It was found that, when using small amounts of solvent, a two-phase system, consisting of an agueous phase (containing the catalyst as a solid) and an organic phase, is formed at least at the end of the reaction. In addition, it was found that the reaction can be carried out with very small amounts of solvent (less than 40 wt.% calculated with respect to benzaldehyde) so that high production capacities can be achieved. Moreover, a simple recovery procedure can be accomplished.

Examples of suitable solvents are lower alcohols such as methanol and ethanol. Furthermore, it was found that the reaction can be carried out without a separate solvent being used. In that case, only a dispersion medium is present in the reaction mixture. The reaction then takes place in a two-phase system from the beginning.

In principle, any liquid not completely miscible with benzaldehyde or dibenzylamine can be used as the dispersion medium. Water is preferably used as the dispersion medium. The advantage of this is that, at the end of the hydrogenation, a 2-phase system is obtained with almost all the dibenzylamine formed present in the organic phase. The aqueous phase, in which the catalyst is included and in which virtually no dibenzylamine or other organic components are present, can, if required, be recirculated as such. The aqueous phase can also be easily removed, after recovery of the catalyst without this causing any great environmental damage.

Small amounts of solvent or dispersion medium are quite adequate to enable the reaction to run properly and to counter substantial deactivation of the catalyst. Because only small amounts of solvent or dispersion medium are necessary, an increase in production capacity is achieved. In principle, the quantity of solvent or dispersion medium necessary for the dispersion of the catalyst in the reaction mixture is quite adequate. In practice, this will mean that use is made of a quantity of solvent or dispersion medium in excess of 3 wt.% calculated with respect to the amount of benzaldehyde, preferably 4-40 wt.%, in particular 5-20 wt.%.

The benzaldehyde used and the dibenzylamine obtained can optionally be substituted in the nucleus, for example by one or more halogens, alkyl groups with 1-4 carbon atoms, or alkoxy groups with 1-4 carbon atoms.

The temperature at which the reaction is carried out can vary within a wide range and generally lies between 60 and 150°C, in particular between 70 and 130°C. The reaction is preferably carried out at a temperature between 80 and 120°C because a higher selectivity can then be achieved.

The pressure at which the reaction is carried out is not critical, either, and will generally lie between the vapour pressure of the reaction mixture, which is determined largely by the vapour pressure of NH₃ at the reaction temperature, and 20 MPa, more in particular between 2 and 10 MPa.

The molar ratio of NH₃ to benzaldehyde which is used is generally higher than 0.4. Surprisingly, the upper limit was not found to be critical.In practice, the molar ratio of NH₃ to benzaldehyde will generally lie between 0.5 and 1.0, preferably between 0.55 and 0.7. The optimum ratio is determined by the extent to which benzylamine and tribenzylamine are obtained as byproduct and is dependent on the type of palladium catalyst. At a lower NH₃-benzaldehyde ratio, somewhat more tribenzylamine is formed; at a higher NH₃-benzaldehyde ratio, somewhat more benzylamine is formed. The optimum ratio for a given catalyst is easily established by empirical methods.

As the catalyst, a catalyst containing palladium is used; use is preferably made of a palladium-carbon catalyst. The amount of palladium with respect to carbon is generally 5-10 wt.% calculated with respect to the total amount of palladium and carbon. In practice starting from a catalyst containing 5 wt.% palladium, mostly an amount of catalyst to be used of 0.05-0.2 wt.% calculated with respect to the amount of benzaldehyde will be sufficient to reach good results. If a catalyst with a different palladium-carbon weight ratio is used, an equivalent amount of catalyst will be used. The optimum amount of catalyst can easily be determined by a person skilled in the art.

### Example I

41.9 g benzaldehyde (395.3 mmol), 20.1 g CH₃OH, 0.08 g 5% palladium/carbon (Degussa; E10 R/W 50% H₂O w/w) and 3.8g NH₃ (223.5 mmol) were successively introduced into an inertised reactor with a capacity of 160 ml provided with a turbine agitator. The reactor was then pressurized to 70 bar (using hydrogen). Next, the temperature was raised to 110°C and the pressure in the reactor increased to 85 bar using hydrogen. After approximately 15 minutes no more hydrogen was absorbed. Next, the reactor contents were cooled to room temperature and the reactor was then depressurized and flushed with N₂. After the catalyst had been filtered off, the reaction mixture was found to be a 2-phase system which, however, is homogenized after the addition of the CH₃OH which was used for the flushing of the autoclave. The homogenous reaction mixture obtained in this way was analyzed by means of gas chromatography. All the benzaldehyde had been completely converted. The selectivity to dibenzylamine was 95.1%. In addition, small amounts of benzylalcohol (sel. approx. 1.8%) and tribenzylamine were formed.

### Examples II - IX

Examples II - IX were carried out in the same way as example I. The results have been reproduced in table 1.

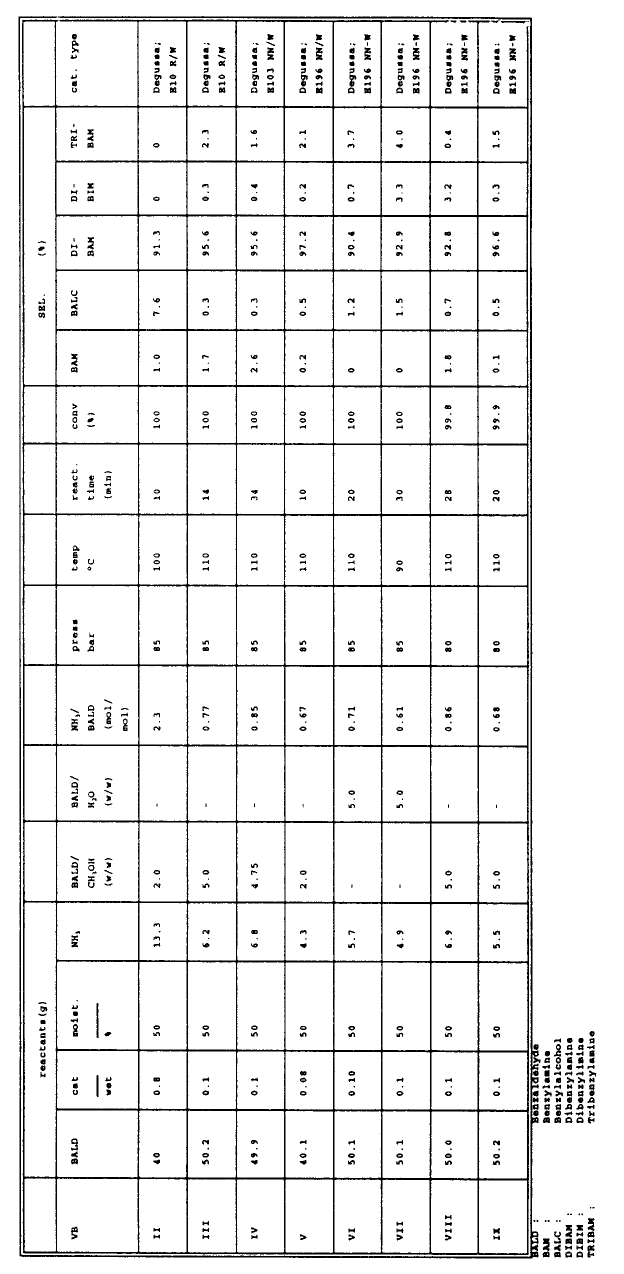

### Example X

37.22 kg BALD (0.35 kmol) and 7.51 kg H₂O were successively introduced into an inertised Buss loop reactor with a capacity of 50 litres. Next, the circulation pump was started and 0.0744 kg 5% palladium/carbon (Degussa E196 NN-WW; 50% H₂O) was added. The reactor was pressurized with five bar hydrogen and preheated to 60°C. Next, 4.34 kg NH₃ (0.255 kmol) was added, after which the temperature was increased to 90°C and the pressure in the reactor was raised to 80 bar using hydrogen. After 45 minutes no further H₂ adsorption took place. The reaction was then continued for a further 30 minutes, after which the reactor contents were cooled (approximately 40°C) and the reactor was depressurized and purged with N₂.
After the catalyst had been filtered off, the reaction mixture was found to be a 2-phase system. After the separation of the two liquid phases the organic phase was analyzed by means of gas chromatography. All the benzaldehyde had been completely converted. The selectivity to dibenzylamine was to 91.4%. In addition, small amounts of benzylalcohol (sel. approx. 0.6%), benzylamine (sel. approx. 0.5%), dibenzylimine (sel. approx. 0.1%) and tribenzylamine (sel. approx. 3.7%) were formed.

### Example XI

40.74 kg BALD (0.384 kmol) and 4.1 kg H₂O. were successively introduced into an inertised Buss loop reactor with a capacity of 50 litres. The circulation pump was then started and 0.0815 kg 5% palladium/carbon (Degussa E196 NN-W; 50% H₂O) was added.
The trial was further carried out along the same lines as example X, except for the fact that 4.76 kg NH₃ (0,280) kmol) was added. Gas chromatography analysis of the organic phase showed that all the benzaldehyde had been converted. The selectivity to dibenzylamine was 92.5%. In addition, small amounts of benzylalcohol (sel. approx. 0.4%), benzylamine (sel. approx. 0.9%), dibenzylimine (sel. approx. 0.1%) and tribenzylamine (sel. approx. 2.4%) were formed.

### Example XII

42 kg BALD (0.396 kmol) and 3 kg H₂O were succesively introduced into an intertised Buss-loop reactor with a capacity of 50 liters. The circulation pump was then started and 0.084 kg 5% Pd/C (Degussa E196 NN-W; 50% H₂O) was added.

The reactor was pressurized with five bar hydrogen and preheated to 60°C. Next, 49 kg NH₃ (0.288 kmol) was added, after which the temperature was increased to 90°C and the pressure raised to 80 bar using hydrogen. The trial was further carried out along the same lines as example X. After separation of the two liquid phases, 28.3 kg of an organic phase and 11.6 kg of a water phase were obtained.
Analysis of both phases gave the following compositions:

| | Organic phase (wt.%) | H₂O-phase (wt.%) |
|---|---|---|
| Benzaldehyde | 0 | 0 |
| Benzylalcohol | 1.5 | 0.2 |
| Benzylamine | 1.7 | 0.5 |
| Dibenzylamine | 94.5 | 0.1 |
| Dibenzylimine | 0.3 | - |
| Tribenzylamine | 1.0 | - |
| H₂O | 0.9 | - |
| NH₃ | 0.05 | 11.3 |

So all the benzaldehyde had been converted and practically no dibenzylamine is present in the waterphase. The yield of DIBAM based on the amount present in the organic phase was 92.5%.

## Claims

1. Process for the preparation of dibenzylamine through hydrogenation of benzaldehyde in the presence of ammonia and a catalyst containing palladium, characterized in that the reaction is performed in the presence of a solvent or dispersion medium in an amount of 3-60 wt.% calculated with respect to benzaldehyde.

2. Process according to claim 1, characterized in that the solvent or dispersion medium is present in an amount of 5-20 wt.%.

3. Process according to claim 1 or 2, characterized in that a palladium-carbon catalyst is used as the catalyst.

4. Process according to any of claims 1-3, characterized in that water is used as the dispersion medium .

5. Process according to any of claims 1-4, characterized in that the reaction is carried out at a temperature between 80 and 120°C.

## Patentansprüche

1. Verfahren zur Herstellung von Dibenzylamin durch Hydrieren von Benzaldehyd in Gegenwart von Ammoniak und einem Katalysator, der Palladium enthält, dadurch gekennzeichnet, daß die Umsetzung in Gegenwart eines Lösungsmittels oder Dispersionsmediums in einer Menge von 3-60 Gewichtsprozent, berechnet in bezug auf Benzaldehyd, ausgeführt wird.

2. Verfahren nach Anspruch 1, dadurch gekennzeichnet, daß das Lösungsmittel oder Dispersionsmedium in einer Menge von 5-20 Gewichtsprozent vorliegt.

3. Verfahren nach Anspruch 1 oder 2, dadurch gekennzeichnet, daß als Katalysator ein Palladium-Kohlenstoff-Katalysator verwendet wird.

4. Verfahren nach einem der Ansprüche 1-3, dadurch gekennzeichnet, daß Wasser als das Dispersionsmedium verwendet wird.

5. Verfahren nach einem der Ansprüche 1-4, dadurch gekennzeichnet, daß die Umsetzung bei einer Temperatur zwischen 80 und 120°C ausgeführt wird.

## Revendications

1. Procédé pour la préparation de dibenzylamine par hydrogénation de benzaldéhyde en présence d'ammoniac et d'un catalyseur contenant du palladium, caractérisé en ce que la réaction est réalisée en présence d'un solvant ou d'un milieu de dispersion à raison de 3 à 60% en poids calculé par rapport au benzaldéhyde.

2. Procédé selon la revendication 1, caractérisé en ce que le solvant ou le milieu de dispersion est présent à raison de 5 à 20% en poids.

3. Procédé selon la revendication 1 ou 2, caractérisé en ce qu'un catalyseur palladium/carbone est utilisé en tant que catalyseur.

4. Procédé selon l'une quelconque des revendications 1 à 3, caractérisé en ce que l'eau est utilisée en tant que milieu de dispersion.

5. Procédé selon l'une quelconque des revendications 1 à 4, caractérisé en ce que la réaction est effectuée à une température comprise entre 80 et 120°C.
